# EUROPEAN PATENT SPECIFICATION

(11) **EP 2 514 916 B1**
(45) Date of publication and mention of the grant of the patent: **18.09.2013**
(21) Application number: 12164493.4
(22) Date of filing: 17.04.2012
(51) Int. Cl.: E02D 1/02, E21B 47/022, G01N 33/24

(54) **Apparatus and method for monitoring geotechnical and structural parameters of soils, rocks and structures in general, in holes having different inclinations or on surfaces having different spatial orientations**
Gerät und Verfahren um geotechnische und strukturelle Boden-, Gestein- oder Konstruktionsparametern zu kontrollieren, in Löchern mit verschiedenen Neigungen oder auf Flächen mit verschiedenen räumlichen Orientierungen
Appareil et méthode pour surveiller des paramètres géotechniques et structuraux de sols, roches et structures en général, dans des trous de différentes inclinaisons ou sur des surfaces ayant diverses orientations spatiales

(30) Priority: 18.04.2011 IT GE20110045
(43) Date of publication of application: 24.10.2012
(73) Proprietor: C.S.G. S.R.L., 15010 Ricaldone (AL) (IT)
(72) Inventor: Foglino, Luigi, I-15010 Ricaldone (AL) (IT); Foglino, Sara, I-15010 Ricaldone (AL) (IT); Foglino, Valentina, I-15010 Ricaldone (AL) (IT)
(74) Representative: Karaghiosoff, Giorgio Alessandro

(56) References cited:
- EP-A1- 2 256 291
- EP-A2- 1 664 486
- FR-A1- 2 497 870
- FR-A1- 2 730 005
- US-A- 2 851 785

## Description

The present invention relates to an apparatus for monitoring geotechnical and structural parameters of soils, rocks and structures in general, comprising at least two or more rigid housing elements, at least one sensor for at least one parameter being mounted in at least one of them.

The at least two or more rigid housing elements are arranged in succession one after the other along a predetermined line or a predetermined axis, a deformable connection element being interposed between each rigid housing element and the one immediately next to it, and providing at least one degree of freedom in the relative motion of the two rigid elements connected thereby.

The at least one sensor communicates with means for collecting output signals therefrom, which may be mounted in the apparatus or in a separate remote station.

A variety of apparatus are known in the art for monitoring geotechnical and structural parameters, as for example the apparatus disclosed in document EP 1664486 by the Applicant hereof, which discloses an apparatus according to the preamble of claim 1.

Therefore, prior art apparatus appear like a succession of rigid elements arranged along an axis, and alternating with deformable elements, i.e. somewhat in the form of a deformable column, articulated at the ends of the rigid elements, for safe adhesion to the soil in the seat, i.e. the hole, where the monitoring apparatus is placed.

Such prior art apparatus currently have a multiparameter monitoring operation, i.e. the sensors in the monitoring apparatus are chemical sensors, temperature sensors, pressure and piezometric level sensors, electric potential meters and tilt sensors.

Nevertheless, these sensors are sensitive to the position of the individual rigid element in which they are located, whereby they are strongly affected by the initial position of the rigid element, as the initial state thereof is the value that the sensor takes as a reference for recording the relevant parameter to be monitored.

Particularly concerning tilt sensors, the reference frame of the sensor coincides with the reference frame of the rigid element, i.e. the x, y, z axes of the sensor coincide with those of the rigid element and the sensor records any change along the axes in case of displacement of the rigid element.

Nevertheless, tilt sensors have short ranges, i.e. do not measure beyond a given angle, that can be variable, in certain sensor models, to ensure an accuracy compatible with the type of application; the measurement system will become inaccurate if this range is exceeded.

This drawback is particularly undesirable if monitoring has to be performed on surfaces or at attitudes that are already inclined to the vertical plane, as any tilt change exceeding the range of the tilt sensor would not be indicated. This faulty operation also occurs when the surface to be monitored, in addition to being inclined to the vertical plane, has a number of tilt changes, like in the case of a tunnel or the profile of a rocky crest, where each rigid element of the apparatus has a different inclination.

In an attempt to obviate this drawback, prior art apparatus have the tilt sensor mounted to a support that has been already inclined to the angle of the area to be monitored, but this requires full knowledge of the area of interest, and also prevents reuse of the apparatus for holes having different inclinations.

Therefore, there exists a yet unfulfilled need for an apparatus for monitoring geotechnical and structural parameters of soils, rocks and structures in general which, using relatively simple and inexpensive arrangements, allows independent monitoring of the conditions of such structure, i.e. by providing a flexible and simple to use apparatus that can adapt its own detection characteristics to field operating conditions.

The invention fulfills the above objects by the provision of an apparatus as described hereinbefore, in which each of the at least two or more rigid housing elements contains means for adjusting the operating conditions of the at least one sensor to the operating conditions of the corresponding housing element, thereby allowing the output signal of the sensor to be adjusted to a predetermined reference value, which falls within the measurement range of the sensor in the initial installation state of the apparatus.

Particularly, the at least one sensor is a position sensor and the adjustment means consist of a sensor support element, which support element is mounted in the rigid housing element in such a manner as to be displaceable with at least one degree of freedom relative to the rigid housing element, control means being provided for controlling the displacement of the support element to one of a plurality of positions and for locking such position.

Therefore, additional adjustment means are provided in the rigid elements, which allow recording by the sensor/s in the rigid element, and allow the operation of the sensor/s to be adapted to a value that falls within the range of the sensor/s by adjusting the output signal thereof.

Advantageously, such adjustment is performed by setting the initial conditions of the sensor/s, i.e. by acting upon the values of the parameter to be monitored captured by the sensor/s at the start of recording.

The initial conditions are preferably set to a zero value, which corresponds to the zero value of the output signal of the at least one sensor.

Since the changes in the parameters to be monitored do not generally exceed the measurement range of the sensor, this important feature allows the zero of the output signal of the sensor to be set to field conditions, which means that the reference frame of the sensor is changed according to field conditions and not according to the position of each rigid housing element that contains the sensor.

This allows monitoring of changes that in prior art apparatus might fall out of the range of values that the sensor can record, especially when the values under the conditions of the area to be monitored are different from the reference values at which the sensors are set.

It shall be noted that the apparatus of the present invention may have any number of sensors, sensors of different types, i.e. of any type known in the art, held in the same rigid housing element or in different rigid housing elements. The housing elements may have the same number of sensors, or each of them may have different numbers or types of sensors.

According to this embodiment of the apparatus of the invention, the at least one sensor is a position sensor and the adjustment means consist of a support element for the at least one sensor, which is mounted in the rigid housing element in such a manner as to be displaceable with at least one degree of freedom relative to the rigid housing element, the rigid element also containing control means for controlling the displacement of the support element to one of a plurality of positions and for locking such position, which means may be actuated externally to and remotely from the rigid housing element.

Therefore, the movement of the support element may be remotely actuated, preferably using electric control means which communicate with the above described output data collecting means, which are in turn connected to the sensors, namely with the one or more sensor support elements.

Advantageously control means are provided for controlling the displacement of the support element to one of a plurality of positions and for locking such position, which means are contained in said housing element and can be accessed from the outside through an access aperture having a removable sealing cover.

Therefore, in addition to controlling the movement of the sensor support element by remote electronic means, one may also directly, even manually adjust the position of the support element and hence the operation of the sensor/s.

Control means are provided for controlling the adjustment operation of the adjustment means for the at least one sensor. These control means may detect the output signal to check whether the initial conditions of the sensor have been properly set according to the initial conditions of the parameter to be monitored. Instead of or in combination with the above, the control means may directly act upon the operating condition that has been set, e.g. on the proper position of the sensor support element.

The first solution may be preferably used when the control means consist of electronic means, whereas the second solution is appropriate when the control means consist of mechanical and/or manual adjustment means.

In a preferred variant embodiment, the sensor in the rigid housing element is a tilt meter and the support element is mounted in the rigid element to swing about a predetermined axis, to change the relative orientation of the tilt sensor to one axis of the rigid housing element.

As described above, the angle of the support element that is swingingly mounted in the rigid housing element can be adjusted using control means, manually or by remotely controllable means, such as motorized means or the like.

With the above described features, even when the area to be monitored consists of holes or attitudes or surfaces having a non-zero inclination to the vertical plane, the amplitude of tilt is recorded as a zero value of the sensor due to the swinging motion of the support element, which is swung by an angle equal and opposite to the swinging angle covered by the vertical axis of the rigid housing element.

In an improvement of the apparatus of the present invention, each rigid housing element consists of a rigid tubular casing with at least one aperture on the shell wall for access from the outside and adjustment of the support element in the casing, whereas removable sealing means are provided for such aperture.

Particularly, the aperture sealing means consist of a tubular sleeve, which is mounted to slide along the outer surface of the tubular casing, and is coaxial thereto, with O-rings being provided at the ends of the sleeve for the sleeve to slide in sealing fashion.

In addition to the advantage of an easy sliding motion, the sleeve can also facilitate access for adjustment of sensor operation, which may be performed, as described above, by adjusting the support element.

According to a preferred variant embodiment, the tilt sensor is mounted to a support element, which consists of a support plate that swings in the tubular housing element about a diametrical axis, whereas the reference axis of the tilt sensor is oriented toward the longitudinal axis of the tubular rigid element, and the angular position between the reference axis of the tilt sensor and the axis of the tubular element is changed by swinging the support plate about the diametrical swinging axis, the plate being adapted to be angularly moved on a shaft coaxial to the axis and to be locked on said axis by fastener means, or means for stopping it in the selected angular position.

This important feature allows operation in any spatial condition, from 0° to 360°, while maintaining the characteristics of the tilt sensor unchanged, with the directivity of the axes of the sensor being maintained, e.g. by simple mechanical fastening of the sensor support element, using screws of the like.

Advantageously, each rigid housing element or at least some of said rigid housing elements, have means for removable anchoring and/or locking them relative to the soil and/or the structure to be monitored.

Such anchor means consist of any prior art means that can lock the apparatus for monitoring geotechnical and structural parameters to the area being monitored, and particularly may consist of deformable "packer" elements, which are known in the art and/or disclosed in EP 1664486.

The apparatus of the present invention is particularly efficient when used in pipes or bore holes having a non-zero inclination to a vertical axis of an absolute reference frame. As used herein, the term absolute reference frame is intended to designate a reference frame oriented with the x, y and z axes integral to those of the earth-gravity reference frame, in which the vertical axis, i.e. the z axis is directed toward the earth center and perpendicular to the earth surface.

Alternatively, the apparatus of the present invention may be used along walls, slopes, tunnel vaults, bridges, arches or the like, with said apparatus being fixed to such structures via fastener means such as brackets or the like.

The use of the apparatus of the present invention, in the form of a modular apparatus comprising rigid elements having different attitudes, to a sub-horizontal attitude, allows differential analysis of three-dimensional soil displacements relative to initial starting conditions, which is particularly useful when monitoring the effects of tunneling in urban environments.

A further application of the apparatus of the present invention is for sub-horizontal installations in trenches.

This configuration is particularly advantageous and suitable when monitoring is performed on shallow soil movements or in the analysis of flowslide, debris flows or soil slip initiation conditions.

These phenomena occur at a shallow level, and prior art monitoring apparatus cannot be laid horizontally, just below the surface, unlike the adjustment means of the apparatus of the present invention, which can set the tilt sensor such that measurements with sub-horizontal attitudes will not fall out of the measurement range of the sensor.

Particularly, the multiparameter monitoring apparatus (for monitoring tilt, acceleration, fluid pressure, temperature, etc.) of the present invention allows direct geotechnical monitoring in the unstable deposit, along the entire section at risk and in a continuous manner, to detect precursors of mechanisms like debris flows, which can be difficultly sensed by instruments unless at shallow depth.

The present invention also relates to a method of monitoring geotechnical and structural parameters of soils, rocks and structures in general. The method comprises the steps of:
a) placing an apparatus for monitoring geotechnical and structural parameters having the above described features along profiles and/or on attitudes and/or in holes with non-zero inclinations to a vertical axis,
b) adjusting said support element for the reference axes of said at least one tilt sensor to be integral with an absolute reference frame,
c) locking said support element in position,
d) capturing the values of the parameter to be monitored.

Advantageously, the step b) may be carried out by causing the sensor support element to swing by an angle equal and opposite to the angle between the vertical axis of the rigid element and an absolute vertical axis.

The reference frame of the tilt sensor is initially integral with the reference frame of the rigid element, but this does not allow all measurements to be made, and especially prevents measurements in holes or along walls that are inclined to the vertical axis of an absolute reference frame.

Therefore, by swinging the sensor support element the reference frame of the sensor is caused to become integral with the absolute reference frame, such that all soil movements can be recorded by the sensor without falling out of range.

Such adjustment of the support element may be carried out on site, in the above described manner, otherwise the swinging angle to be covered by the support element may be determined beforehand.

Thus, if the installation attitude is known beforehand, the various rigid elements of the monitoring apparatus may be assembled in the laboratory, otherwise they may be assembled with a "0" attitude, according to the pre-oriented fixed frame, and be adjusted during installation according to the inclination required to remain within the measurement range of the sensors.

This will afford high flexibility and easy use, as well as monitoring along possibly irregular surfaces, which might not be monitored using conventional fixed tilt sensors, by combining the modular linking and measurement range adaptability requirements.

In one embodiment of the method of the present invention, the steps b) and c) may be carried out through said control means that can be actuated externally to and remotely from the rigid housing element.

Alternatively, the steps b) and c) may be carried out by the control means in said housing element, which can be accessed from the outside through said access aperture. Here, a step is provided before the step b), in which said aperture is opened by sliding said tubular sleeve, and a further step is provided after the step c), in which said aperture is closed by sliding said tubular sleeve.

These and other features and advantages of the invention will be more apparent from the following description of a few embodiments shown in the accompanying drawings, in which:
Fig. 1 shows the modular configuration of both the apparatus of the present invention and prior art apparatus;
Fig. 2a is a view of the apparatus of the present invention according to a preferred embodiment;
FIG. 2b shows a cross-section of an element as taken along a vertical plane of the apparatus as show in Figure 2a;
Fig. 3a shows the detail of the sensor housing area of Figure 2a;
FIG. 3b shows a cross-section of an element as taken along a vertical plane of the apparatus as show in Figure 3a;
Figs. 4a to 4d show different installations of the apparatus of the present invention.

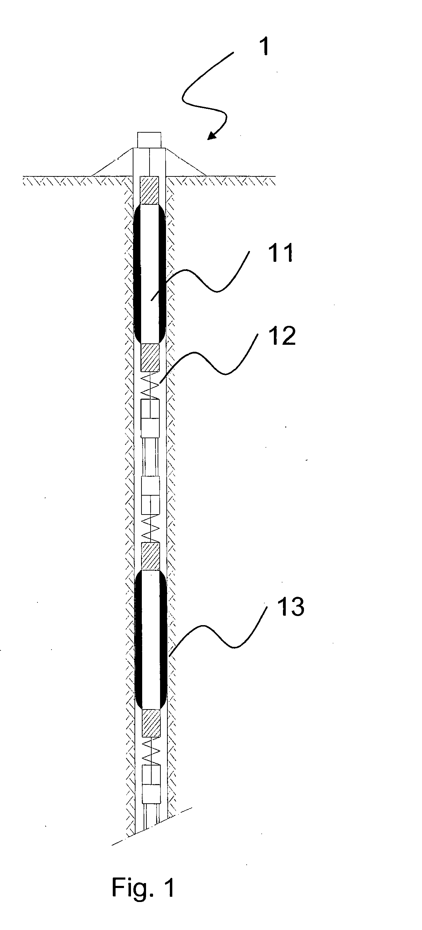

Figure 1 shows the modular construction of the monitoring apparatus of the present invention, with such apparatus being composed of a plurality of rigid housing elements 11, at least one sensor for at least one parameter being mounted in at least one of them. The rigid housing elements 11 are arranged in succession one after the other along a predetermined line or a predetermined axis, and a deformable connection element 12 is interposed between each rigid element 11 and the one immediately next to it.

The presence of the deformable element 12 is designed to allow each rigid element 11 at least one degree of freedom.

The apparatus of the present invention, according to a preferred embodiment, is of the 2D type, which means that each rigid element 11 has 2 degrees of freedom, and particularly the rigid element 11 may move over a plane x-y relative to an absolute reference frame integral with the earth-gravity reference frame.

The joint, i.e. the deformable connection element 12, may be further designed to allow each rigid element 3 degrees of freedom, i.e. changes along the x, y and z axes.

The selection of either of the 2D and 3D base configurations is made according to the purpose required according to the medium to be monitored and to various technical, economic and construction requirements.

Namely, for instance for landslide tilt monitoring, which does not require measurement of the changes in the distances between rigid elements 11, but only of deflection between one rigid element and the other, a 2D joint should be used, which has a less expensive construction while affording the same effectiveness.

When both measurements of deflection and monitoring of the distance between rigid elements are required, e.g. in case of failures due to soil stabilization or in case of underground collapses, a 3D joint must be used, which allows measurement of the z axis in addition to the x and y axes.

The configuration as shown in Figure 1 is the construction of prior art monitoring apparatus, which is extensively described in document EP 1664486 by the Applicant hereof.

Particularly, Figure 1 also shows the removable anchoring and/or locking means 13 as described in the above mentioned document, which allow adhesion of the monitoring apparatus 1 to the soil and/or the walls of the structure to be monitored.

Preferably, each rigid element 11 is a multiparameter element, which means that it has various types of sensors, i.e. chemical sensors, temperature sensors, pressure and piezometric level sensors, electric potential meters ad well as position and tilt sensors.

Each sensor communicates with means for collecting output signals therefrom, which may be mounted in the apparatus, held within the rigid elements 11 or in a separate remote station.

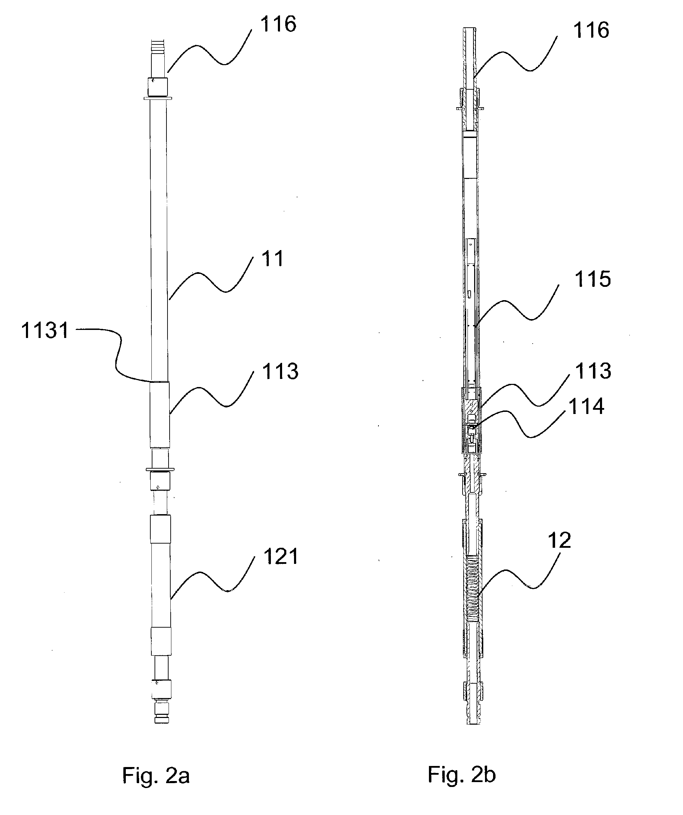

Nevertheless, the above configuration is affected by the above mentioned drawbacks and disadvantages, and Figures 2a to 3b show the monitoring apparatus of the present invention according to a preferred embodiment, which solves such drawbacks.

Generally, in the monitoring apparatus of the present invention, each of the rigid housing elements 11 contains means 111 for adjusting the operating conditions of the sensors 112 to the operating conditions of the corresponding housing element 11, thereby allowing the output signal of the sensor 112 to be adjusted to a predetermined reference value, which falls within the measurement range of the sensor 112 in the initial installation state of the apparatus.

Preferably, the operating conditions of each sensor 112 are adjusted by adjusting the initial conditions thereof, the initial conditions being the parameter values as detected by each sensor 112 at the start of detection.

Particularly referring to Figures 2a and 2b, the monitoring apparatus of the present invention is composed of a plurality of rigid housing elements 11 in the form of tubular cylindrical casings, which tubular cylindrical casing 11 houses a sensor 112 connected to an electronic data collection and processing board 115 which may in turn be connected, through a terminal portion 116 of said casing 11, to a remote unit, not shown.

The cylindrical casing 11 is connected to a deformable element 12 which consists, in this particular case, of a spring that allows the casing 11 to move with two degrees of freedom.

The spring 12 may be covered by a reinforced braid 121, which prevents the spring 12 from being crushed at its sides during the movement of the tubular casing 11, and hence restricting such movement of the casing 11.

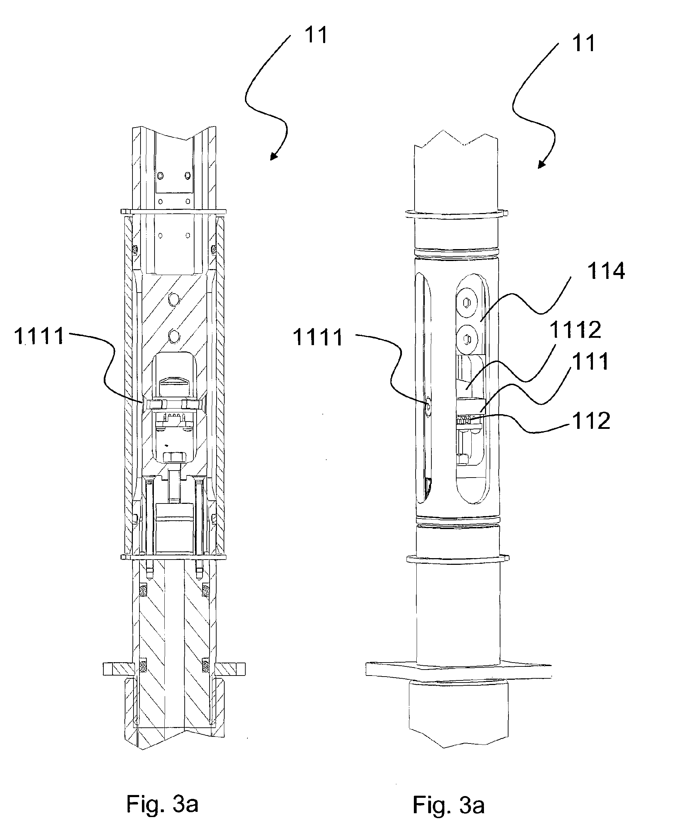

Particularly in Figures 3a and 3b, a detail of the monitoring apparatus of the present invention is shown, in which the sensor is a position sensor and the adjustment means consist of a support element 111 for said sensor 112.

The support element 111 is mounted in the tubular housing casing 11, to be movable with at least one degree of freedom relative to the reference frame thereof, and control means 1111 are provided for controlling the displacement of the support element 11 to one of a plurality of positions.

The control means 1111 are also designed to lock the predetermined position and can be accessed from the outside through an access aperture 114 having a removable sealing cover 113.

Instead of or in combination with the above, the control means may be arranged to be actuated externally to and/or remotely from the rigid housing element 11 and preferably to be electronically controlled through the electronic control board 115.

In addition to the control means 1111, control means 1112 may be provided for controlling the movement of the support element 111, which are adapted to allow a check of the output signal of the sensor 112 and/or the operating condition that has been set.

Particularly referring to Figures 3a and 3b, the sensor 112 is a tilt sensor 112 mounted to a swinging plate 111 which acts as a support element therefor and allows the position of the sensor 112 to be changed, and thus change its reference frame.

Thus, the sensor 112 initially has a reference frame integral with the one of the tubular casing 11, i.e. with the axes oriented in the same direction. If the reference frame of the casing 11 is not integral with the earth-gravity reference frame, i.e. if the apparatus is positioned with some inclination to the vertical axis, then the swinging plate 111 allows the orientation of the sensor 112 to be rotated for its reference frame to be integral with the absolute earth-gravity reference frame.

In the particular case of Figures 3a and 3b, the support plate 111 rotates about the x axis, although it can also rotate about the y and z axes.

Furthermore, the sensor rotation control means consist of a spherical level 1112 which can check whether the "0" output value of the tilt sensor 112 actually derives from a match of the reference frame of the sensor 112 with the absolute reference frame, and in this particular case whether the z axis of the sensor 112 is coincident with the vertical axis of the absolute reference frame.

The control means 1111 may be accessed for adjustment of the tilt of the support plate 111 through the aperture 114 formed on the shell wall of the rigid tubular casing 11.

The aperture 114 also has removable sealing means 113 (as shown in Figure 2a) consisting of a tubular sleeve 113, which is mounted to slide along the lateral surface of the rigid casing 11, and whose sealing action is ensured by two O-rings 1131 or the like at each of the ends of the sleeve 113.

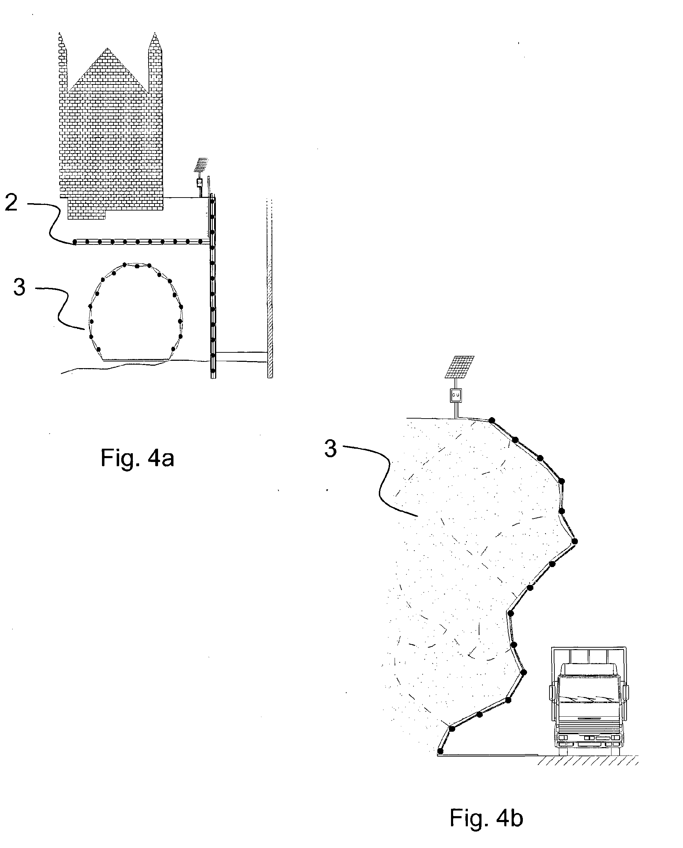

Figures 4a to 4d show possible ways of use of the monitoring apparatus of the present invention.

The above described features make such apparatus particularly efficient in pipes or bore holes having a non-zero inclination to a vertical axis of an absolute reference frame, and even in horizontal holes 2 like in the case of Figure 4a.

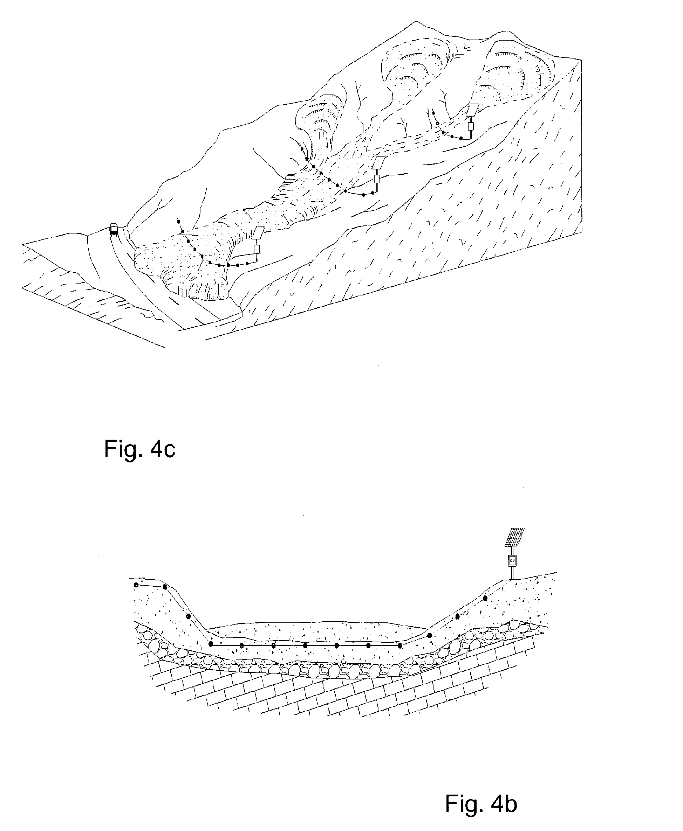

Furthermore, since each tilt sensor 112 may be adjusted according to the positioning arrangement of the casing 11 in which it is held, and in a different manner for each casing, the monitoring apparatus of the present invention may be advantageously used on walls 3 with profiles including many direction and inclination changes, like in Figure 4b or in the tunnel of Figure 4a.

A further application of the apparatus of the present invention is for sub-horizontal installations in trenches, as shown in Figures 4c and 4d.

This configuration is particularly advantageous and suitable when monitoring is performed on shallow soil movements or in the analysis of flowslide, debris flows or soil slip initiation conditions.

These phenomena occur at a shallow level, and prior art monitoring apparatus cannot be laid horizontally, just below the surface, unlike the adjustment means of the apparatus of the present invention, which can set the tilt sensor such that measurements with sub-horizontal attitudes will not fall out of the measurement range of the sensor.

Particularly, the multiparameter monitoring apparatus (for monitoring tilt, acceleration, fluid pressure, temperature, etc.) of the present invention allows direct geotechnical monitoring in the unstable deposit, along the entire section at risk and in a continuous manner, to detect precursors of mechanisms like debris flows, which can be difficultly sensed by instruments unless at shallow depth.

## Claims

1. An apparatus (1) for monitoring geotechnical and structural parameters of soils, rocks and structures in general, comprising at least two or more rigid housing elements (11), at least one sensor (112) for at least one parameter, the sensor being mounted in at least one of the housing elements,
which at least two or more rigid housing elements (11) are arranged in succession one after the other along a predetermined line or a predetermined axis, a deformable connection element (12) being interposed between each rigid housing element and the one immediately next to it, which deformable connection element (12) provides at least one degree of freedom in the relative motion of the two rigid elements (11) connected thereby,
said at least one sensor (112) communicating with means (115) for collecting output signals therefrom, which collection means may be mounted in the apparatus (1) or in a separate remote station,
**characterized in that**
each of said at least two or more rigid housing elements (11) contains means (111) for adjusting the operating conditions of said at least one sensor (112) to the operating conditions of the corresponding housing element (11), thereby allowing the output signal of the sensor (112) to be adjusted to a predetermined reference value, which falls within the measurement range of the sensor in the initial installation state of the apparatus,
said at least one sensor (112) being a position sensor and said adjustment means (111) consisting of a support element (111) for said at least one sensor (112), which support element is mounted in said rigid housing element (11) in such a manner as to be displaceable with at least one degree of freedom relative to said rigid housing element, and wherein control means are provided for controlling the displacement of said support element to one of a plurality of positions and for locking said position.

2. An apparatus (1) for monitoring geotechnical and structural parameters as claimed in claim 1, wherein said control means are contained in said housing element and can be accessed from the outside through an access aperture (114) having a removable sealing cover (113).

3. An apparatus (1) as claimed in claim 1, wherein said control means are arranged to be actuated externally to and remotely from the rigid housing element (11).

4. An apparatus as claimed in one or more of the preceding claims, wherein the sensor (112) is a tilt meter and said support element (111) is swingingly mounted in the rigid element (11), to change the relative orientation of the tilt sensor to one axis of the rigid housing element.

5. An apparatus as claimed in one or more of the preceding claims, wherein each rigid housing element (11) consists of a rigid tubular casing, which rigid tubular casing has at least one aperture (114) on the shell wall for access from the outside and adjustment of said support element (111), removable sealing means (113) being provided for such aperture.

6. An apparatus as claimed in one or more of the preceding claims, wherein said sealing means consist of a tubular sleeve (113) which is mounted to slide along the outer surface of said tubular casing (11) and coaxial therewith,
O-rings or the like (1113) being provided at each of the ends of said sleeve.

7. An apparatus as claimed in one or more of the preceding claims 4 to 6, wherein the tilt sensor (112) is mounted to a support plate (111) that swings in the tubular housing element (11) about a diametrical axis, whereas the reference axis of the tilt sensor (112) is oriented toward the longitudinal axis of the tubular rigid element, the angular position between said reference axis of the tilt sensor and the axis of the tubular element (11) being changed by swinging the support plate (111) about said diametrical swinging axis, said plate (111) being adapted to be angularly moved on a shaft coaxial to said axis and to be locked on said axis by fastener means, or means for stopping it in the selected angular position.

8. An apparatus as claimed in one or more of the preceding claims, wherein each rigid housing element (11) or at least some of said rigid housing elements, have means for removable anchoring and/or locking them relative to the soil and/or the structure to be monitored (13).

9. A use of the apparatus as claimed in one or more of the preceding claims 1 to 8 in pipes or bore holes having a non-zero inclination to a vertical axis of an absolute reference frame.

10. A use of the apparatus as claimed in one or more of the preceding claims 1 to 8 along walls, slopes, tunnel vaults, bridges, arches or the like, with said apparatus being fixed to such walls, slopes, tunnel vaults, bridges, arches or the like via fastener means such as brackets or the like.

11. A use of the apparatus as claimed in one or more of the preceding claims 1 to 8 for sub-horizontal installations in trenches.

12. A method of monitoring geotechnical and structural parameters of soils, rocks and structures in general,
**characterized in that** it comprises the steps of:
a) placing an apparatus for monitoring geotechnical and structural parameters as claimed in one or more of claims 1 to 8, along profiles and/or on attitudes and/or in holes with non-zero inclinations to a vertical axis,
b) adjusting said support element for the reference axes of said at least one tilt sensor to be integral with an absolute reference frame,
c) locking said support element in position,
d) capturing the values of the parameter to be monitored.

13. A method as claimed in claim 12, wherein the step b) is carried out by causing said support element to swing by an angle equal and opposite to the angle between the vertical axis of said rigid element and an absolute vertical axis.

14. A method as claimed in claim 12 or 13, wherein the steps b) and c) are carried out by said control means in said housing element, which can be accessed from the outside through said access aperture,
a step being provided before the step b), in which said aperture is opened by sliding said tubular sleeve, and a further step being provided after the step c), in which said aperture is closed by sliding said tubular sleeve.

## Patentansprüche

1. Ein Gerät (1) zur Überwachung geotechnischer und struktureller Boden-, Gestein- oder Konstruktionsparametern, bestehend aus
- mindestens zwei oder mehr biegesteifen Gehäuseelementen (111),
- aus mindestens einem Sensor (112) für mindestens einen der Parameter, wobei der Sensor in mindestens einen der Gehäuseelemente eingebaut ist,
- die mindestens zwei oder mehreren biegesteifen Gehäuseelementen (111) eine nach dem anderen in Reihe angeordnet sind, entlang einer vorgegebenen Linie oder einer vorgegebenen Achse,
- aus einem deformierbaren Verbindungselement (12), das zwischen jedem biegesteifen Gehäuseelement und dem einen unmittelbar benachbart zu diesem eingefügt ist, welches deformierbare Verbindungselement (12) mindestens ein Freiheitsgrad in der Relativbewegung der beiden biegesteifen Elemente (111) zur Verfügung stellt, die dadurch miteinander verbunden sind,
- wobei die genannten mindestens einen der Sensoren (112) mit Mitteln (115) kommunizieren, um Ausgabesignale daraus zu sammeln, welche Sammelmittel in dem Gerät (1) oder in einer getrennten Fernbedienungsstation eingebaut sind,
**dadurch gekennzeichnet,**
**dass**, jeder der genannten mindestens zwei oder mehreren biegesteifen Gehäuseelemente (111) Mittel (111) aufweist, um die Bedienbedingungen der genannten mindestens einer der Sensoren (112), zu den Betriebsbedingungen des korrespondierenden Gehäuseelementes (111) einzustellen, die es dabei erlauben, dass das Ausgangssignal des Sensors (112) an einen vorgegebenen Referenzwert angepasst ist, das innerhalb des Messbereiches des Sensors in der Werkseinstellung des Gerätes liegt,
**dass** der genannte mindestens eine Sensor (112) ein Positionssensor ist, und die genannten Einstellungsmittel (111) ein Unterstützungselement (111) für den mindestens einen Sensor (112) umfassen, welche Unterstützungselement in dem genannten biegesteifen Gehäuseelement (111) angebracht ist, in einer solchen Art beweglich ist, dass diese mit mindestens einem Grad Bewegungsfreiheit relativ zu dem genannten biegesteifen Gehäuseelement einstellbar ist und wobei Kontrollmittel zur Verfügung gestellt sind, um die Ausrichtung des genannten Unterstützungselementes zu kontrollieren zu einer von einer Vielzahl von Positionen und um die genannte Position zu sperren.

2. Ein Gerät (1) zur Überwachung von geotechnischen und strukturellen Parametern, wie in Patentanspruch 1 beansprucht,
**dadurch gekennzeichnet,**
**dass** die genannten Kontrollmittel in dem genannten Gehäuseelement eingebaut sind, die von außen durch eine Zugangseinrichtung (114) zugänglich sind, die eine bewegliche Verschlussabdeckung (113) aufweist.

3. Ein Gerät (1), wie in Patentanspruch 1 beansprucht,
**dadurch gekennzeichnet,**
**dass** die genannten Kontrollmittel derart angeordnet sind, dass diese extern durch und fernbedient von den biegesteifen Geräteelemente (111) in Betrieb gesetzt sind.

4. Ein Gerät (1), wie in einem oder mehreren der vorgenannten Ansprüche beansprucht,
**dadurch gekennzeichnet,**
**dass** der Sensor (112) ein Kippsensor ist und dass das genannte Unterstützungselement (111) schwingend in dem biegesteifen Element (111) angebracht ist, um die relative Orientierung des Kippsensors zu einer Achse des biegesteifen Gehäuseelementes zu ändern.

5. Ein Gerät (1), wie in einem oder mehreren der vorgenannten Ansprüche beansprucht,
**dadurch gekennzeichnet,**
**dass** jedes biegesteife Gehäuseelement (111) aus einem biegesteifen röhrenförmigen Überzug gebildet ist, welcher biegesteife röhrenförmige Überzug mindestens eine Apparatur (114) auf der Mantelfläche hat, durch die ein Zugang von der Außenseite und eine Einstellmöglichkeit des genannten Unterstützungselementes (111) geschaffen ist, wobei bewegliche Verschlussmittel (113) für solche Apparaturen zur Verfügung gestellt sind.

6. Ein Gerät wie in einem oder mehreren der vorgenannten Ansprüche beansprucht,
**dadurch gekennzeichnet,**
**dass** die genannten Verschlussmittel aus einer röhrenförmigen Hülse (113) bestehen, die derart befestigt ist, dass diese entlang der Außenfläche des genannten röhrenförmigen Überzuges (111) und zu dieser co-axial gleitet,
**dass** O-Ringe oder dergleichen (1113) an jedem der Enden der genannten Hülse vorhanden sind.

7. Ein Gerät, wie in einem oder mehreren der vorgenannten Ansprüche 4 bis 6 beansprucht,
**dadurch gekennzeichnet,**
**dass** der Kippsensor (112) an einer Unterstützungsplatte (111) befestigt ist, die in dem röhrenförmigen Gehäuseelement (111) um eine diasoziale Achse schwingt, wobei die Referenzachse des Kippsensors (112) zu der Längsachse des röhrenförmigen biegesteifen Elementes ausgerichtet ist, die schräge Position zwischen der genannten Referenzachse des Kippsensors und der Achse des röhrenförmigen Elementes (111) durch Schwingungen der Unterstützungsplatte (111) um die genannte diasoziale Schwingungsachse geändert ist, wobei die genannte Platte (111) derart ausgerichtet ist, dass diese schräg auf einem Schaft bewegbar ist, der coaxial zu der genannten Achse und Feststellmittel auf der genannten Achse gesperrt ist oder Mittel zur Arretierung dieser in der ausgewählten, geneigten Stellung aufweist.

8. Ein Gerät wie in einem oder mehreren der vorgenannten Ansprüche beansprucht,
**dadurch gekennzeichnet,**
**dass** jedes biegesteife Gehäuseelement (111) oder mindestens einige der genannten biegesteifen Gehäuseelemente Mittel haben, um diese relativ zu dem Boden und/oder zu der Struktur, lösbar zu verankern und/oder zu sperren die zu überwachen ist (13).

9. Eine Anwendung des Gerätes, wie in einem oder mehreren der vorgenannten Ansprüche 1 bis 8 beansprucht, in Röhren- oder Bohrlöchern, die eine nicht Nullneigung in Bezug auf eine vertikale Achse eines absoluten Referenzrahmen aufweisen.

10. Eine Anwendung des Gerätes, wie in einem oder mehreren der vorgenannten Ansprüche 1 bis 8 beansprucht, entlang Wänden, Abhängen, Tunnelwölbungen, Brücken, Bogengewölben oder dergleichen, in dem das genannte Gerät auf solchen Wänden, Abhängen, Tunnelgewölben, Brücken, Bogengewölben oder dergleichen via Befestigungsmitteln, wie Schellen oder dergleichen arretiert ist.

11. Anwendung des Gerätes, wie in einem oder mehreren der vorgenannten Ansprüche 1 bis 8 beansprucht, zur zwischenhorizontalen Befestigung in Ankergräben.

12. Ein Verfahren zur Überwachung geotechnischer und struktureller Boden-, Gesteins- und Konstruktionsparameter,
**dadurch gekennzeichnet,**
**dass** es die nachfolgenden Schritte umfasst:
a) Anordnen eines Gerätes zur Überwachung von geotechnischen und strukturellen Parametern, wie in einem oder mehreren der Patentansprüche 1 bis 8 beansprucht, entlang Profilen und/oder an Erhöhungen und/oder in Löchern mit einer Nichtnullneigung zu einer vertikalen Achse,
b) Ausrichten des genannten Unterstützungselementes für die Referenzachse des genannten mindestens einen Kippsensors der Bestandteil mit einem absoluten Referenzrahmen ist,
c) Arretieren des genannten Unterstützungselementes in einer Position,
d) Ermitteln der Werte der Parameter, die zu überwachen sind.

13. Verfahren, wie in Patentanspruch 12 beansprucht,
**dadurch gekennzeichnet,**
**dass** der Schritt b) dadurch ausgeführt ist, dass das Unterstützungselement um einen Winkel in Schwingung versetzt ist, der gleich und gegenüberliegend zu einem Winkel zwischen der vertikalen Achse des genannten biegesteifen Elementes und einer absoluten vertikalen Achse liegt.

14. Ein Verfahren, wie in Patentanspruch 12 oder 13 beansprucht,
**dadurch gekennzeichnet,**
**dass** die Schritte b) und c) durch die genannten Kontrollmittel in dem genannten Gehäuseelement ausgeführt sind, das von der Außenseite durch die genannte Zugangsapparatur zugänglich ist,
**dass** ein Schritt vor dem Schritt b) vorgesehen ist, bei dem die genannten Apparatur durch Entlanggleiten der genannten röhrenförmigen Hülse vorgesehen ist und ein weiterer Schritt nach dem Schritt c) vorgesehen ist, bei dem die genannten Apparatur durch Entlanggleiten der genannten röhrenförmigen Hülse geschlossen ist.

## Revendications

1. Appareil (1) pour surveiller des paramètres géotechniques et structurels des sols, des roches et des structures en général, comprenant au moins deux éléments de logement rigides (11) ou plus, au moins un capteur (112) pour au moins un paramètre, le capteur étant monté dans au moins l'un des éléments de logement,
lesquelles au moins deux éléments de logement rigides (11) ou plus sont agencés en succession, l'un après l'autre, le long d'une ligne prédéterminée ou d'un axe prédéterminé, un élément de raccordement déformable (12) étant intercalé entre chaque élément de logement rigide et l'un immédiatement à côté de ce dernier, lequel élément de raccordement déformable (12) fourni au moins un degré de liberté dans le mouvement relatif des deux éléments rigides (11) raccordés ainsi,
ledit au moins un capteur (112) communiquant avec des moyens (115) pour collecter des signaux de sortie provenant de ce dernier, lesquels moyens de collecte peuvent être montés dans l'appareil (1) ou dans une station à distance séparée,
**caractérisé en ce que** :
chacun desdits au moins deux éléments de logement rigides (11) ou plus contient des moyens (111) pour ajuster les conditions de fonctionnement dudit au moins un capteur (112) par rapport aux conditions de fonctionnement de l'élément de logement (11) correspondant, permettant ainsi d'ajuster le signal de sortie du capteur (112) par rapport à une valeur de référence prédéterminée, qui se trouve dans la plage de mesure du capteur dans l'état d'installation initial de l'appareil,
ledit au moins un capteur (112) étant un capteur de position et lesdits moyens d'ajustement (111) se composant d'un élément de support (111) pour ledit au moins un capteur (112), lequel élément de support est monté dans ledit élément de logement rigide (11) afin d'être déplaçable avec au moins un degré de liberté par rapport audit élément de logement rigide, et dans lequel des moyens de commande sont prévus pour commander le déplacement dudit élément de support dans l'une d'une pluralité de positions et pour bloquer ladite position.

2. Appareil (1) pour surveiller des paramètres géotechniques et structurels selon la revendication 1, dans lequel lesdits moyens de commande sont contenus dans ledit élément de logement et auxquels on peut avoir accès depuis l'extérieur par une ouverture d'accès (114) ayant un couvercle d'étanchéité amovible (113).

3. Appareil (1) selon la revendication 1, dans lequel lesdits moyens de commande sont agencés pour être actionnés extérieurement et à distance de l'élément de logement rigide (11).

4. Appareil selon l'une ou plusieurs des revendications précédentes, dans lequel le capteur (112) est un indicateur de pente et ledit élément de support (111) est monté de manière oscillante dans l'élément rigide (11) pour modifier l'orientation relative de l'indicateur de pente par rapport à un axe de l'élément de logement rigide.

5. Appareil selon l'une ou plusieurs des revendications précédentes, dans lequel chaque élément de logement rigide (11) se compose d'un boîtier tubulaire rigide, lequel boîtier tubulaire rigide a au moins une ouverture (114) sur la paroi de coque pour l'accès depuis l'extérieur et l'ajustement dudit élément de support (111), des moyens d'étanchéité amovibles (113) étant prévus pour une telle ouverture.

6. Appareil selon l'une ou plusieurs des revendications précédentes, dans lequel lesdits moyens d'étanchéité se composent d'un manchon tubulaire (113) qui est monté pour coulisser le long de la surface externe dudit boîtier tubulaire (11) et est coaxial avec ce dernier,
des joints toriques ou similaires (1113) étant prévus à chacune des extrémités dudit manchon.

7. Appareil selon l'une ou plusieurs des revendications 4 à 6, dans lequel l'indicateur de pente (112) est monté sur une plaque de support (111) qui oscille dans l'élément de logement tubulaire (11) autour d'un axe diamétral, alors que l'axe de référence de l'indicateur de pente (112) est orienté vers l'axe longitudinal de l'élément rigide tubulaire, la position angulaire entre ledit axe de référence de l'indicateur de pente et l'axe de l'élément tubulaire (11) étant modifiée en faisant osciller la plaque de support (111) autour dudit axe oscillant diamétral, ladite plaque (111) étant adaptée pour être déplacée de manière angulaire sur un arbre coaxial audit axe et être bloquée sur ledit axe par des moyens de fixation, ou des moyens pour l'arrêter dans la position angulaire sélectionnée.

8. Appareil selon l'une ou plusieurs des revendications précédentes, dans lequel chaque élément de logement rigide (11) ou au moins certains desdits éléments de logement rigides, a (ont) des moyens pour l'ancrer (les ancrer) et/ou le (les) bloquer de manière amovible par rapport au sol et/ou à la structure à surveiller (13).

9. Utilisation de l'appareil selon l'une ou plusieurs des revendications 1 à 8, dans des tuyaux ou des sondages ayant une inclinaison non nulle par rapport à un axe vertical d'un cadre de référence absolu.

10. Utilisation de l'appareil selon l'une ou plusieurs des revendications 1 à 8, le long de parois, pentes, voutes de tunnel, ponts, arches ou similaires, avec ledit appareil qui est fixé sur de telles parois, pentes, voutes de tunnel, ponts, arches ou similaires via des moyens de fixation tels que des consoles ou similaires.

11. Utilisation de l'appareil selon une ou plusieurs des revendications 1 à 8 pour des installations de faible inclinaison dans des tranchées.

12. Procédé pour surveiller des paramètres géotechniques et structurels des sols, des roches et des structures en général,
**caractérisé en ce qu'**il comprend les étapes consistant à :
a) placer un appareil pour surveiller des paramètres géotechniques et structurels selon une ou plusieurs des revendications 1 à 8, le long de profils et/ou orientations et/ou dans des trous avec des inclinaisons non nulles par rapport à un axe vertical,
b) ajuster ledit élément de support pour que les axes de référence dudit au moins un indicateur de pente soient intégrés avec un cadre de référence absolu,
c) bloquer ledit élément de support en position,
d) prendre les valeurs du paramètre à surveiller.

13. Procédé selon la revendication 12, dans lequel l'étape b) est réalisée en amenant ledit élément de support à osciller selon un angle égal et opposé à l'angle entre l'axe vertical dudit élément rigide et un axe vertical absolu.

14. Procédé selon la revendication 12 ou 13, dans lequel les étapes b) et c) sont réalisées par lesdits moyens de commande dans ledit élément de logement, auxquels on peut avoir accès depuis l'extérieur par ladite ouverture d'accès,
une étape étant prévue avant l'étape b), dans laquelle ladite ouverture est ouverte en faisant coulisser ledit manchon tubulaire, et une étape supplémentaire étant prévue après l'étape c), dans laquelle ladite ouverture est fermée en faisant coulisser ledit manchon tubulaire.
